(19) Europäisches Patentamt
European Patent Office
Office européen des brevets

(11) Publication number : **0 323 846 B1**

# (12) EUROPEAN PATENT SPECIFICATION

(45) Date of publication of patent specification : **08.04.92 Bulletin 92/15**

(51) Int. Cl.$^5$ : **C07C 315/00**

(21) Application number : **89100156.2**

(22) Date of filing : **05.01.89**

## (54) Process of preparing thiamphenicol.

(30) Priority : **08.01.88 JP 2228/88**
**24.11.88 JP 296897/88**

(43) Date of publication of application :
**12.07.89 Bulletin 89/28**

(45) Publication of the grant of the patent :
**08.04.92 Bulletin 92/15**

(84) Designated Contracting States :
**DE ES FR IT**

(56) References cited :
**EP-A- 0 224 902**
**JP-A-49 125 327**
**US-A- 2 759 976**

(73) Proprietor : **NIPPON ZEON CO., LTD.**
**6-1, Marunouchi 2-chome**
**Chiyoda-ku Tokyo 100 (JP)**

(72) Inventor : **Tanaka, Kimiaki**
**3-3, Inamuragasaki-5-chome**
**Kamakura-shi (JP)**
Inventor : **Yagi, Hiroshi**
**15-33, Isogo-2-chome**
**Isogo-ku Yokohama (JP)**
Inventor : **Mitsuda, Yasuhiro**
**26-3-406, Kajiwara-2-chome**
**Kamakura-shi (JP)**

(74) Representative : **Vossius & Partner**
**Siebertstrasse 4 P.O. Box 86 07 67**
**W-8000 München 86 (DE)**

EP 0 323 846 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an efficient process of preparing thiamphenicol.

Thiamphenicol is used as a broad spectrum antibacterial agent. A process described in Japanese Patent Application KOKAI (the term "KOKAI" as used herein refers to an unexamined application which was laid open for public inspection) No. 125327/1974 is known to be a simple method for synthesis of thiamphenicol. This process comprises using chloramphenicol represented by formula [III] below as starting material, reducing the nitro group to the amino group followed by diazotization, then reacting the resulting diazonium compound with a methylthio metal salt and oxidizing the methylthio group to prepare thiamphenicol.

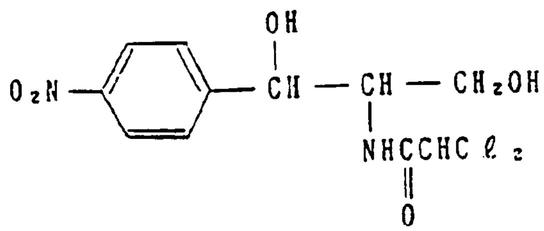

( III )

Thiamphenicol can be obtained by this process in relatively few steps which, however, have various drawbacks.

That is, the process involves the following problems: (1) selectivity of the reduction of the nitro group in the starting material is poor, (2) the obtained diazonium compound precipitates and hence operations for isolating it are necessary, (3) the diazonium compound must be dried so that there is a danger of explosion, (4) side reactions occur between the dichloro moiety of the dichloroacetyl group and the methylthio metal salt during the formation of the methylthio group to reduce the selectivity, resulting in a lower yield of the product.

Thus the technical problem underlying the present invention is to develop a process for preparing thiamphenicol which eliminates the foregoing drawbacks. The solution of this problem is evident from claim 1. It has been found that by using the compound of formula [I] later described containing an amino group on the aliphatic side chain which is not dichloroacetylated, the diazonium compound obtained goes into solution in the reaction mixture; thus, the steps of isolation and drying can be omitted. Accordingly, the formation of the methylthio group can be carried out with high selectivity so that thiamphenicol can be obtained efficiently in a simple operation.

The present invention relates to a process of preparing thiamphenicol represented by formula [II] described below, which comprises diazotizing an aromatic amino group in the compound represented by formula [I] described below, reacting the resulting diazonium compound with a methylthio metal salt, dichloroacetylating the aliphatic amino group of the reaction product and then oxidizing the methylthio group; alternatively, oxidizing the methylthio group followed by dichloroacetylation.

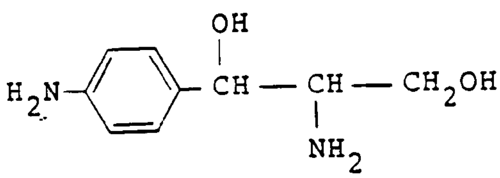

[I]

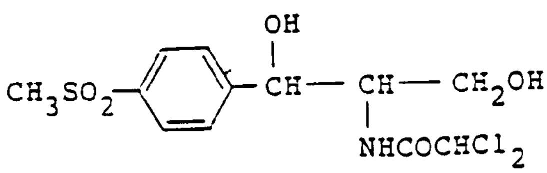

[II]

The starting compound used in the present invention is D-threo-1-p-aminophenyl-2-aminopropane-1,3-diol. It has the formula [I]. This compound can be readily obtained in high selectivity by reducing the nitro group of the compound represented by formula [IV]

(D-threo-1-p-nitrophenyl-2-aminopropane-1,3-diol), which is a chloramphenicol precursor, in a conventional manner.

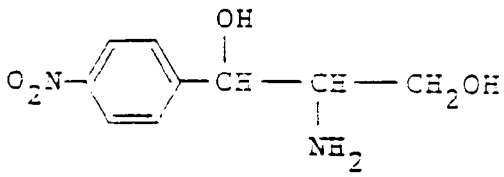

[IV]

According to the present invention, the aromatic amino group of the starting compound [I] is diazotized to prepare the diazonium compound. Diazotization may be performed in a conventional manner by reacting compound [I] with sodium nitrite in an acidic solvent at a low temperature. For example, compound [I] is reacted with sodium nitrite in an acidic solvent with stirring for 1 to 2 hours while maintaining the temperature at 0 to 10°C. Diazotization occurs selectively on the aromatic amino group and therefore, it is unnecessary to protect the aliphatic amino group.

In the present invention, the diazonium compound obtained goes into solution in the reaction mixture. Thus, the reaction with the methylthio metal salt at the following step can be carried out in the same reaction mixture. That is, the process of the present invention does not require separate steps for isolating and drying the diazonium compound that were necessary in the prior art process.

Besides, the danger of explosion accompanied by drying the unstable diazonium compound can be avoided.

Next, the formed diazonium compound is reacted with the metal salt of methylmercaptane. Examples of suitable metal salts of methylmercaptane are salts of alkali metals such as sodium, potassium.

The reaction temperature is generally between -10 and 50°C, preferably between 0 and 40°C. The reaction time is 0.5 to 2 hours.

As described above, the diazonium compound whose aliphatic amino group had been dichloroacetylated was used in the prior art process and therefore, side reactions occurred between the metal salt of methylmercaptane and the dichloro moiety. However, according to the process of the present invention, the aliphatic amino group in the diazonium compound contains no dichloro moiety so that there is no danger of side reactions.

The reaction product is obtained as a precipitate. The mother liquor containing the precipitate may be used in the dichloroacetylation at the following step as it is, but the mother liquor is preferably filtered to obtain the reaction product as a filter cake, the product is dissolved in an appropriate solvent by adding an acid thereto and the solution is evaporated. The residue is used for the dichloroacetylation; the yield and purity are improved.

Such a solvent may be a polar organic solvent inert to the reaction and examples include lower alcohols such as methanol, ethanol, propanol; ethers such as tetrahydrofuran, dioxane, ethylene glycol dimethyl ether. Of these, lower alcohols were preferably used. Examples of suitable acids are inorganic acids such as hydrochloric acid, sulfuric acid, nitric acid, carbonic acid, etc., and organic acids such as formic acid, acetic acid, propionic acid. Inorganic acids are preferred.

Then, the obtained reaction product is dichloroacetylated in an appropriate solvent. Examples of the solvents are lower alcohols such as methanol, ethanol, propanol.

The dichloroacetylating agent used is not particularly limited as far as it is capable of reacting with the aliphatic amino group to form an acid amide bond. Examples of the dichloroacetylating agent are dichloroacetic acid esters such as methyl dichloroacetate, ethyl dichloroacetate; dichloroacetic acid halides such as dichloroacetic chloride, dichloroacetic bromide.

The reaction temperature is generally between 0 and 70°C but in case that dichloroacetatic acid esters are used as the acetylating agent, the temperature is preferably between 30 and 70°C. Further in the case of using dichloroacetic acid halides, the temperature is preferably between 0 and 10°C. The reaction time is generally 1 to 2 hours.

In the present invention, the dichloroacetylated product is ordinarily dissolved in the system. Therefore, the dichloroacetylated product is isolated by extraction with a solvent and then oxidized. For the oxidization, oxidizing agents such as hydrogen peroxide, ozone, a halogen, metal oxides such as a permanganate, chromic acid, ruthenium tetroxide, organic peroxides such as perbenzoic acid, metachloroperbenzoic acid, monoperoxyphthalic acid, performic acid, peracetic acid, trifluoroperacetic acid, may be used. When oxidizing aids are used in combination in this case, better results are achieved. Typical examples of such aids are acids such as acetic acid, glacial acid, hydrochloric acid, sulfuric acid, oxides of metals such as tungsten, molybdenum, titanium, chromium, acids such as tungstic acid, molybdic acid, titanic acid, chromic acid, bichromic acid, or alkali metal salts or alkaline earth metal salts thereof, and heteropoly acids such as phosphotungstic acid, silicotungstic acid, phosphomolybdic acid, or alkali metal salts thereof.

The reaction conditions are not generally set forth but may vary depending upon the kind of oxidation to

be adopted; for example, in the method using hydrogen peroxide, the reaction temperature is between 20 and 80°C, preferably between 40 and 70°C and the reaction time is 3 to 6 hours.

Further in the present invention, reaction conditions for the case that the order of dichloroacetylation and oxidation is reversed are also similar to the aforesaid two types of reaction conditions.

After completion of the reaction, the objective product is isolated from the reaction mixture in a conventional manner by means of recrystallization, column chromatography. Thus, thiamphenicol can be obtained in high purity.

According to the present invention, thiamphenicol can be obtained more efficiently when compared to the prior art techniques.

Hereafter the present invention is described in more detail by referring to the examples below. In the examples and comparative example, parts and % are all by weight unless otherwise indicated.

Example 1

To a solution of 51.5 g of D-threo-1-p-aminophenyl-2-aminopropane-1,3-diol in 110 g of 36% hydrochloric acid were added 51 g of 41% sodium nitrite aqueous solution at about 0°C over an hour with stirring. The mixture was then reacted for further 2 hours to give a solution of D-threo-1-p-diazophenyl-2-aminopropane-1,3-diol. The solution was added to 540 g of a 15% aqueous solution of methylthio sodium salt. The mixture was reacted for an hour by heating to 40°C with stirring. Further 360 g of methanol and 120 g of methyl dichloroacetate were added thereto. The mixture was heated to about 50°C and reacted for an hour.

The reaction product was extracted with about 300 ml of ethyl acetate. After concentrating to dryness, 150 ml of acetic acid were added to the residue and 66 g of 35% hydrogen peroxide were added to the mixture over 30 minutes while keeping at about 60°C. This temperature was maintained for further 4 hours. The reaction solution was concentrated. The concentrate was dissolved in water. The solution was allowed to stand under cooling to give crude thiamphenicol. The crude product was recrystallized from water to give 46 g of thiamphenicol. This product showed a melting point of 165°C, $[\alpha]_D$ of +12.8° (c=2, ethanol). IR spectra, NMR spectra, mass spectra and elemental analysis were all identical with those of the standard compound.

Comparative Example 1

Chloramphenicol, 65 g, was dissolved in 300 ml of methanol and 2.5 g of 5% palladium-carbon catalyst and 40 ml of conc. hydrochloric acid were added to the solution. While keeping below 30°C, catalytic reduction was performed. After cooling, the catalyst was filtered and the filtrate was concentrated. Crystallization was performed from isopropanol to give 60 g of D-threo-1-p-aminophenyl-2-dichloroacetamidopropane-1,3-diol. This product was dissolved in 240 ml of water and 24 ml of hydrochloric acid. The resulting solution was kept at about 0°C. Then 150 ml of an aqueous solution containing 16 g of sodium nitrite were added. D-threo-1-p-diazonium chloride phenyl-2-dichloroacetamidopropane-1,3-diol precipitated and was filtered to give 49.8 g.

To a solution of 11 g of methylthio sodium salt in 73 ml of ethanol were added 438 ml of benzene. While keeping the solution at about 0°C, 49.8 g of the diazonium salt described above were added to the solution over 30 minutes. After stirring at the temperature for further 30 minutes, the mixture was heated to 40°C and stirred for an hour. After the formed sodium chloride was removed, the reaction mixture was concentrated to dryness and, 584 ml of ethyl acetate and 219 ml of water were added to the residue. After extracting D-threo-1-p-methylmercaptophenyl-2-dichloroacetamidopropane-1,3-diol with ethyl acetate, the extract was concentrated to dryness. To the solid were added 219 ml of acetic acid. The mixture was kept at about 60°C and 70 ml of 35% hydrogen peroxide were added thereto over 30 minutes. The mixture was kept at the temperature for further 3 to 4 hours. After the reaction solution was concentrated, the concentrate was dissolved in water and the solution was allowed to cool to give crude thiamphenicol. The crude product was recrystallized from water to give 8.2 g of thiamphenicol.

Example 2

A solution of D-threo-1-p-diazophenyl-2-aminopropane-1,3-diol was obtained in a manner similar to Example 1. The solution was added to 540 g of a 15% aqueous solution of methylthio sodium salt. While stirring, the mixture was heated to 40°C and reacted for an hour to give a reaction solution containing 51.5 g of a precipitate. After 115 g of acetic acid were added to the reaction solution, the mixture was heated to about 60°C and 66 g of hydrogen peroxide were added thereto over 30 minutes. The mixture was reacted for further 4 hours to give a solution of D-threo-1-p-methylsulfonyl-2-aminopropane-1,3-diol. After further concentrating and evaporating to dryness, 360 g of methanol were added to the residue. The mixture was filtered to remove inorganic salts and the methanolic solution was obtained. To the solution were added 48 g of methyl dichloroacetate. The mixture was reacted for an hour by heating to about 50°C and then concentrated. The concentrate was dissolved in water and the solution was allowed to cool to give crude thiamphenicol. The crude product was recrystallized from water to give 46 g of thiamphenicol.

Example 3

A solution of D-threo-1-p-diazophenyl-2-aminopropane-1,3-diol was obtained in a manner similar to Example 1. The solution was added to 540 g of a 15% aqueous solution of methylthio sodium salt. After stirring for an hour, the reaction solution was filtered. To the obtained cake were added 360 g of methanol and 29.4 g of 36% hydrochloric acid. The mixture was heated to 40°C to give a solution of D-threo-1-p-methylmercaptophenyl-2-aminopropane-1,3-diol. After the solution was evaporated to dryness, 360 g of methanol were added to dissolve the residue,and 48 g of methyl dichloroacetate. While keeping the pH of the reaction mixture at about 9 with a methanolic solution of potassium hydroxide, the mixture was heated to 50°C and reacted for an hour.

The reaction product was extracted with about 300 ml of ethyl acetate. After concentrating and evaporating to dryness, 150 ml of acetic acid were added to the residue. While keeping at about 60°C, 66 g of 35% hydrogen peroxide were added thereto over 30 minutes. The temperature was maintained for further 4 hours. The reaction solution was concentrated. The concentrate was dissolved in water and the solution was allowed to cool to give crude thiamphenicol. The crude product was recrystallized from water to give 50 g of thiamphenicol.

Example 4

A solution of D-threo-1-p-methylmercaptophenyl-2-aminopropane-1,3-diol was obtained in a manner similar to Example 3. After evaporating the solution to dryness, 360 g of methanol were added to dissolve the residue. Then 48 g of methyl dichloroacetate were added to the solution. While keeping the pH of the reaction mixture to about 9 with a methanolic solution of potassium hydroxide, the mixture was heated to about 50°C and reacted for an hour.

Then, 6 g of tungstic acid were added to the reaction solution. While keeping at about 60°C, 46 g of 35% hydrogen peroxide were added thereto over 30 minutes. The temperature was maintained for further 3 hours. The reaction solution was concentrated. The concentrate was dissolved in water and the solution was allowed to cool to give crude thiamphenicol. The crude product was recrystallized from water to give 51 g of thiamphenicol.

## Claims

1. A process of preparing thiamphenicol represented by formula **[II]** below characterized by:

a step of diazotizing the aromatic amino group of the compound represented by formula **[I]** below;

a step of subsequently reacting a methylthio metal salt with said diazonium compound to convert the amino group into a methylthio group; and,

a combination of a step of dichloroacetylating the aliphatic amino group and a step of oxidizing the methylthio group.

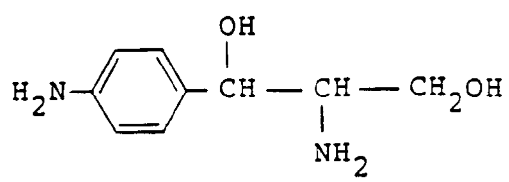

[I]

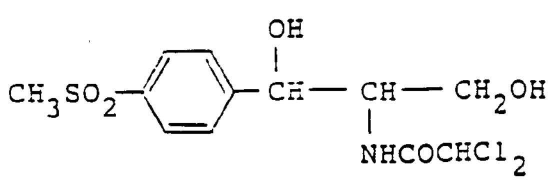

[II]

2. A process of preparing thiamphenicol as claimed in claim 1, wherein said step of dichloroacetylating the aliphatic amino group is followed by the step of oxidizing the methylthio group.

3. A process of preparing thiamphenicol as claimed in claim 1, wherein said step of oxidizing the methylthio group is followed by the step of dichloroacetylating the aliphatic amino group.

4. A process of preparing thiamphenicol as claimed in claim 1, wherein said metal salt of methylmercaptane is an alkali metal salt.

5. A process of preparing thiamphenicol as claimed in claim 1, wherein the reaction product obtained at the step of reacting with the metal salt of methylmercaptane is filtered and the filter cake obtained is dissolved with an acid in a solvent.

6. A process of preparing thiamphenicol as claimed in claim 5, wherein said acid is an inorganic acid or an organic acid.

7. A process of preparing thiamphenicol as claimed in claim 1, wherein a dichloroacetylating agent is selected from an ester and halide of dichloroacetic acid.

8. A process of preparing thiamphenicol as claimed in claim 2 or 3, wherein oxidation of the methylthio group is performed in the presence of an oxidizing agent and an oxidizing aid.

**Patentansprüche**

1. Verfahren zur Herstellung von Thiamphenicol der nachstehenden Formel [II], gekennzeichnet durch:

einen Schritt, bei dem die aromatische Aminogruppe der Verbindung der nachstehenden Formel [I] diazotiert wird;

einen Schritt, bei dem anschließend ein Methylthiometallsalz mit der Diazoniumverbindung umgesetzt wird, wobei die Aminogruppe in eine Methylthiogruppe umgewandelt wird; und

eine Kombination eines Schrittes, bei dem die aliphatische Aminogruppe dichloracetyliert wird und eines Schrittes, bei dem die Methylthiogruppe oxidiert wird.

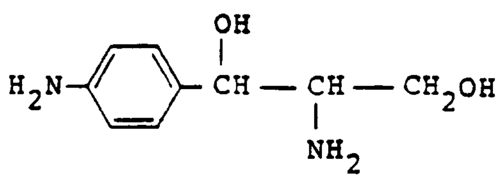

[I]

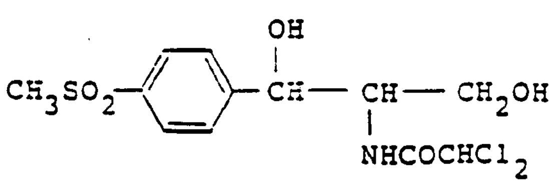

[II]

2. Verfahren zur Herstellung von Thiamphenicol gemäß Anspruch 1, wobei der Schritt der Dichloracetylierung der aliphatischen Aminogruppe von dem Schritt der Oxidierung der Methylthiogruppe gefolgt wird.

3. Verfahren zur Herstellung von Thiamphenicol gemäß Anspruch 1, wobei der Schritt der Oxidierung der Methylthiogruppe vom Schritt der Dichloracetylierung der aliphatischen Aminogruppe gefolgt wird.

4. Verfahren zur Herstellung von Thiamphenicol gemäß Anspruch 1, wobei das Metallsalz von Methylmercaptan ein Alkalimetallsalz ist.

5. Verfahren zur Herstellung von Thiamphenicol gemäß Anspruch 1, wobei das Reaktionsprodukt, das beim Schritt der Umsetzung mit dem Metallsalz von Methylmercaptan erhalten wird, abfiltriert wird und der erhaltene Filterkuchen mit einer Säure in einem Lösungsmittel gelöst wird.

6. Verfahren zur Herstellung von Thiamphenicol gemäß Anspruch 5, wobei die Säure eine anorganische oder eine organische Säure ist.

7. Verfahren zur Herstellung von Thiamphenicol gemäß Anspruch 1, wobei ein Dichloracetylierungsmittel ausgewählt ist aus einem Ester und einem Halogenid der Dichloressigsäure.

8. Verfahren zur Herstellung von Thiamphenicol gemäß Anspruch 2 oder 3, wobei die Oxidation der Methylthiogruppe in Gegenwart eines Oxidationsmittels und eines Oxidationshilfsmittels durchgeführt wird.

## Revendications

1. Procédé pour la préparation de thiamphénicol représenté par la formule [II] ci-après, caractérisé par les étapes consistant :

à diazoter le groupe amino aromatique du composé représenté par la formule [I] ci-après ;

à faire réagir ensuite un sel métallique de méthylmercaptan avec le composé de diazonium pour remplacer le groupe amino par un groupe méthylthio ; et

à combiner une étape de dichloroacétylation du groupe amino aliphatique et une étape d'oxydation du groupe méthylthio.

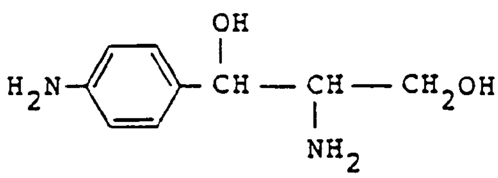

[I]

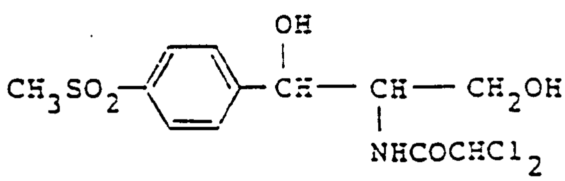

[II]

2. Procédé pour la préparation de thiamphénicol selon la revendication 1, dans lequel ladite étape de dichloroacétylation du groupe amino aliphatique est suivie par l'étape d'oxydation du groupe méthylthio.

3. Procédé pour la préparation de thiamphénicol selon la revendication 1, dans lequel ladite étape d'oxydation du groupe méthylthio est suivie par l'étape de dichloroacétylation du groupe amino aliphatique.

4. Procédé pour la préparation de thiamphénicol selon la revendication 1, dans lequel ledit sel métallique de méthylmercaptan est un sel de métal alcalin.

5. Procédé pour la préparation de thiamphénicol selon la revendication 1, dans lequel le produit réactionnel obtenu dans l'étape de réaction avec le sel métallique de méthylmercaptan est filtré et le gâteau de filtration obtenu est dissous avec un acide dans un solvant.

6. Procédé pour la préparation de thiamphénicol selon la revendication 5, dans lequel ledit acide est un acide minéral ou un acide organique.

7. Procédé pour la préparation de thiamphénicol selon la revendication 1, dans lequel un agent de dichloroacétylation est choisi parmi un ester et un halogénure de l'acide dichloroacétique.

8. Procédé pour la préparation de thiamphénicol selon la revendication 2 ou 3, dans lequel l'oxydation du groupe méthylthio est réalisée en présence d'un agent oxydant et d'un auxiliaire d'oxydation.